# EUROPÄISCHE PATENTANMELDUNG

(11) **EP 4 843 973 A1**
(43) Veröffentlichungstag der Anmeldung: **25.11.2026**
(21) Anmeldenummer: 25178208.2
(22) Anmeldetag: 22.05.2025
(51) Int. Cl.: C07C 5/05, C07C 11/08

(54) **VERFAHREN ZUR BEREITSTELLUNG EINES DAMPFSTROMS DURCH WÄRMEINTEGRATION**

(71) Anmelder: Evonik Oxeno GmbH & Co. KG, 45772 Marl (DE)
(72) Erfinder: PAUL, Niklas, 45770 Marl (DE); RIX, Armin Matthias, 45770 Marl (DE); WESSNER, Lea, 44139 Dortmund (DE); OLDEMEYER, Martin, 45721 Haltern am See (DE); SIX, Tanita Valèrie, 44309 Dortmund (DE); STEENWEG, Tanja, 44139 Dortmund (DE); PEITZ, Stephan, 45739 Oer-Erkenschwick (DE); SIMON, Berno, 45772 Marl (DE); WESSER, Maximilian, 44139 Dortmund (DE); BRUNS, Bastian, 44791 Bochum (DE)
(74) Vertreter: Evonik Patent Association

(57) **Zusammenfassung**

Gegenstand der Erfindung ist ein Verfahren zur Erzeugung eines Dampfstroms, bei dem die bei einer Selektivhydrierung entstehende Energie über einen Wärmeträger W zur Erzeugung eines Dampfstroms genutzt wird. Der Dampfstrom kann dann an anderen Stellen im Verfahren oder an einer anderen Stelle am Produktionsstandort eingesetzt werden.

## Beschreibung

Die vorliegende Erfindung betrifft ein Verfahren zur Erzeugung eines Dampfstroms, bei dem die bei einer Selektivhydrierung entstehende Energie über einen Wärmeträger W zur Erzeugung eines Dampfstroms genutzt wird. Der Dampfstrom kann dann an anderen Stellen im Verfahren oder an einer anderen Stelle am Produktionsstandort eingesetzt werden.

Chemische Produktionsstandorte beherbergen oft eine große Zahl unterschiedlicher chemische Produktionsanlagen, teilweise von völlig unterschiedlichen Unternehmen. Die Produktionsanlagen umfassen häufig neben Reaktoren auch Anlagen oder Anlagenteile zur Aufreinigung des dort produzierten Produkts. Die Aufreinigung findet nicht selten mittels thermischer Trennverfahren statt, beispielweise mittels Destillation.

Chemische Produktionsanlagen benötigen immer wieder hohe Mengen an Energie, sei es für die Durchführung der Reaktion oder die anschließende Aufreinigung mit beispielsweise thermischer Trennung. Die dafür benötigte Energie wird in der Regel über Dampf eingebracht. Chemische Produktionsstandorte verfügen deshalb oft über eine oder mehrere Anlagen zur Erzeugung von Dampf und ein Dampfnetz, mit dem der Dampf zu den Produktionsanlagen transportiert wird.

Der Dampf wird typischerweise aus fossilen Brennstoffen erzeugt. Insofern ist der Einsatz von Dampf aus Dampfnetzen an Produktionsstandorten nicht nur teuer, sondern erzeugt auch führt zu hohen CO₂-Emission.

Die Aufgabe der vorliegenden Erfindung war deshalb die Bereitstellung eines Verfahrens zur Erzeugung eines Dampfstroms, dass nicht nur kostengünstig ist, sondern sich auch durch eine Verringerung der CO₂-Emissionen auszeichnet.

Die Aufgabe wird durch das erfindungsgemäße Verfahren nach Anspruch 1 gelöst. Bevorzugte Ausführungsformen sind in den Unteransprüchen angegeben.

Das erfindungsgemäße Verfahren ist ein Verfahren zur Erzeugung eines Dampfstroms, wobei das Verfahren die folgenden Schritte umfasst
a) Zuführen eines C4-Kohlenwasserstoffstroms, der zumindest 1,3-Butadien, 1-Buten, 2-Buten und Isobutan enthält, und Zuführen eines Wasserstoff-haltigen Gases zu einer Hydrierung, um zumindest einen Teil des enthaltenen 1,3 Butadiens in Anwesenheit eines heterogenen Katalysators zu hydrieren, wobei die Hydrierung in einer Hydrierzone erfolgt, die mindestens einen Reaktor R1 umfasst und wobei aus dem Reaktor R1 ein hydrierter Strom entnommen wird;
b) Kühlen des hydrierten Stroms aus dem Reaktor R1, optional und/oder dem Reaktor R2, in einem Wärmetauscher WT1, wobei Energie in Form von Wärme vom hydrierten Strom auf einen flüssigen oder gasförmigen Wärmeträger W übertragen wird, wodurch ein Wärmeträger W1 entsteht;
c) Verdichten mindestens eines Teils des Wärmeträgers W1, vorzugsweise des gesamten Wärmeträgers W1, wodurch ein verdichteter Wärmeträger W2 entsteht, der einen höheren Druck aufweist als der Wärmeträger W1; und
d) Übertragen von Energie vom verdichteter Wärmeträger W2 auf einen weiteren Wärmeträger D übertragen wird, wodurch der herzustellende Dampfstrom erzeugt wird.

Der Vorteil des erfindungsgemäßen Verfahrens ist, dass der Dampfstrom dadurch erzeugt wird, dass die bei der Selektivhydrierung in Schritt a) entstehende Energie über den Wärmeträger W in Schritt b) eingesammelt und anschließend nach Verdichten (Schritt c)) in Schritt d) auf einen weiteren Wärmeträger D übertragen wird. Dadurch entsteht der gewünschte Dampf. Die Erzeugung erfolgt also mittels Wärmeintegration, d. h. die Energie wird nicht durch einen externen Prozess bereitgestellt, sondern nutzbar gemacht, dass Energie aus einem Prozess, hier der Selektivhydrierung, abgenommen wird. Die Energie zur Kühlung des hydrierten Stroms wird sonst vernichtet, beispielsweise durch den Einsatz eines Kühlmittels.

### Schritt a)

Schritt a) des erfindungsgemäßen Verfahrens betrifft die Selektivhydrierung von 1,3-Butadien in C4-Kohlenwasserstoffströmen. Die Selektivhydrierung zeichnet sich durch das Zuführen eines C4-Kohlenwasserstoffstroms, der zumindest 1,3-Butadien, 1-Buten, 2-Buten und Isobutan enthält, und Zuführen von Wasserstoff zu einer Selektivhydrierung, um zumindest einen Teil des enthaltenen 1,3 Butadiens zu hydrieren aus, wobei die Selektivhydrierung in einer Hydrierzone erfolgt, die mindestens einen Reaktor R1 umfasst und wobei aus dem Reaktor R1 ein hydrierter Strom entnommen wird.

Der in Schritt a) eingesetzte C4-Kohlenwasserstoffstrom ist vorzugsweise ein technisches C4-Kohlenwasserstoffgemische aus katalytischen Crackern oder Steamcrackern. Solche Ströme üblicherweise neben gesättigten (n-Butan, Isobutan) und einfach ungesättigten Verbindungen (Isobuten, 1-Buten, cis- und trans-2-Buten) auch mehrfach ungesättigte Verbindungen wie 1,3-Butadien oder in Spuren Acetylene. In einer bevorzugten Ausführungsform der vorliegenden Erfindung wird ein Crack-C4-Strom als C4-Kohlenwasserstoffstrom in Schritt a) des erfindungsgemäßen Verfahrens eingesetzt.

Ein Crack-C4-Strom kann folgende Zusammensetzung aufweisen:

| | |
|---|---|
| Isobutan | 0,5 bis 5 Gew.-%; |
| Butadien | 25 bis 45 Gew.-%; |
| Isobuten | 17 bis 32 Gew.-%; |
| 1-Buten | 10 bis 25 Gew.-%; |
| 2-Buten | 3 bis 12 Gew.-%; und |
| n-Butan | 5 bis 15 Gew.-%. |

Die Mengen aller Komponenten ergeben jeweils 100 Gew.-%.

Selektivhydrierung bedeutet in diesem Zusammenhang, dass 1,3-Butadien in Anwesenheit der weiteren ungesättigten Verbindungen wie Isobuten oder den Butenen selektiv hydriert wird. Die weiteren C4-Kohlenwasserstoffe mit mindestens einer Doppelbindung werden bei der Selektivhydrierung nicht oder kaum hydriert, sondern bleiben zum überwiegenden Anteil, d. h. bezogen auf ihren ursprünglichen Gesichtsanteil im eingesetzten C4-Kohlenwasserstoffstrom zu >95%, bevorzugt zu >98% unverändert erhalten.

Zur Hydrierung wird ein Wasserstoff-haltiges Gas eingesetzt. Geeignet sind alle Gase oder Gasmischungen, die Wasserstoff in einer Menge enthalten, die eine Hydrierung zulässt. In einer bevorzugten Ausführungsform wird als Wasserstoff-haltiges Gas Wasserstoff eingesetzt.

Der Wasserstoff wird bei der Selektivhydrierung in Schritt a) vorzugsweise im leichten molaren Überschuss im Verhältnis zur zu hydrierenden Mol-Zahl der Doppelbindungen zugegeben, bevorzugt in einer Menge von 1,0 bis 1,5 mol Wasserstoff pro mol Doppelbindung, die hydriert werden soll und besonders bevorzugt in einer Menge von 1,0 bis 1,3 mol Wasserstoff pro mol Doppelbindung, die hydriert werden soll. Durch den maximal geringen Überschuss an Wasserstoff soll verhindert werden, dass durch ein zu großes Überangebot an Wasserstoff Nebenreaktionen auftreten und/oder die anderen Kohlenwasserstoffe abreagieren.

Die Selektivhydrierung in Schritt a) kann in einem breiten Temperaturbereich durchgeführt werden. Die Selektivhydrierung wird vorzugsweise bei einer Temperatur zwischen 20 und 300 °C, weiterhin bevorzugt zwischen 25 und 250 °C und besonders bevorzugt zwischen 30 und 200 °C durchgeführt. Der Druck bei der Selektivhydrierung sollte 50 bar, bevorzugt 30 bar, besonders bevorzugt 20 bar nicht übersteigen. Eine Erhöhung des Drucks über die Einstellung der ausreichenden Löslichkeit von Wasserstoff hinaus hat normalerweise keinen Einfluss auf das Verfahren. Der Druck könnte daher grundsätzlich auch höher sein, würde jedoch einen höheren apparativen Aufwand bedeuten ohne signifikanten Einfluss auf die Reaktion zu haben.

Die erfindungsgemäße Selektivhydrierung kann in der Gasphase oder in der Flüssigphase durchgeführt werden, ist weiterhin bevorzugt aber eine Flüssigphasenhydrierung. Die Temperatur ist in diesem Fall so zu wählen, dass der zu verarbeitende Kohlenwasserstoffstrom in der Flüssigphase vorliegt.

In einer bevorzugten Ausführungsform der vorliegenden Erfindung werden das Wasserstoff-haltiges Gas, vorzugsweise der Wasserstoff und der C4-Kohlenwasserstoffstrom vor dem Zuführen in den Reaktor R1 in einem statischen Mischer vermischt. Statische Mischer sind dem Fachmann bekannt.

Es ist bevorzugt, dass der statische Mischer eine Geometrie aufweist, welche eine Strömung mit einer Reynoldszahl von größer als 100, vorzugsweise von größer als 200, insbesondere von größer als 500, besonders bevorzugt von größer als 900 im statischen Mischer vermittelt. Der Fachmann ist in der Lage die Reynoldszahl basierend auf der Geometrie eines statischen Mischers zu bestimmen. Die allgemeine Formel der Reynoldszahl ist dem Fachmann bekannt.

Es ist weiterhin bevorzugt, dass der statische Mischer eine Geometrie aufweist, welche eine Vermischung des Wasserstoff-haltigen Gases, vorzugsweise des Wasserstoffs und des C4-Kohlenwasserstoffstroms derart vermittelt, dass der Wasserstoff in der flüssigen Phase am Ende des statischen Mischers annähernd in Sättigungskonzentration vorliegt. Der Fachmann ist in der Lage die Sättigungskonzentration in einer flüssigen Phase zu bestimmen.

Unter "annähernd in Sättigungskonzentration" im Rahmen der vorliegenden Erfindung ist gemeint, dass die Konzentration des Wasserstoffs in der flüssigen Phase maximal 15 %, vorzugsweise maximal 10 %, besonders bevorzugt maximal 5 % und ganz besonders bevorzugt maximal 1 % unterhalb der Sättigungskonzentration liegt. Das bedeutet beispielsweise, dass wenn die Sättigungskonzentration des Wasserstoffs in der flüssigen Phase 1 g/L beträgt, dann bedeutet eine Konzentration von 0,85 g/L Wasserstoff in der flüssigen Phase ebenfalls, dass eine Sättigungskonzentration vorliegt.

Daher ist es weiterhin bevorzugt, dass der statische Mischer eine Bauweise ausgewählt aus der Gruppe bestehend aus den Mischertypen Kenics-Mischer, Sulzer SMV-Mischer, Sulzer SMX-Mischer, Fluitec CSE-Mischer und Ross-ISG-Mischer, vorzugsweise eine Kenics-Mischer Bauweise, besitzt. Ein Kenics-Mischer weist eine Geometrie auf, die den Eingangsstrom durch ein spiralförmiges Mischelement radial in Richtung der Rohrwände und zurück zur Mitte leitet. Eine zusätzliche Umkehrung der Fließrichtung und eine Stromaufteilung ergeben sich aus der Kombination von abwechselnd nach links und rechts gedrehten Elementen. Durch diese Fließprofile werden die Eingangsströme gemischt.

Die Selektivhydrierung in Schritt a) erfolgt an einem heterogenen Katalysator. Es können unterschiedliche Katalysatoren eingesetzt werden, vorzugsweise solche, die Palladium und/oder Platin und/oder Silber enthalten.

In einer Ausführungsform der vorliegenden Erfindung wird ein Katalysator auf Basis von Palladium bei der Selektivhydrierung eingesetzt. Vorzugsweise liegt das Palladium auf einem inerten Trägermaterial vor. Bei dem Trägermaterial handelt es sich beispielsweise um Siliciumdioxid, Alumosilikat, Aluminiumoxid, Kieselgel, Aktivkohle, Titandioxid oder Mischungen daraus. Bevorzugt werden Siliciumdioxid, Aluminiumoxid, Titandioxid oder Mischungen daraus als Trägermaterial eingesetzt. Der Katalysator hat eine Palladium-Konzentration, die zwischen 0.01 und 3 %, bezogen auf die Masse des Trägers, liegt. Bevorzugt liegt sie zwischen 0.1 und 1 %, ganz besonders bevorzugt zwischen 0.3 und 0.5 %. Der Katalysator hat eine BET-Oberfläche (bestimmt durch Gasadsorption nach DIN ISO 9277) von 50 bis 400 m²/g, bevorzugt zwischen 100 und 300 m²/g, besonders bevorzugt zwischen 200 und 300 m²/g.

In einer weiteren Ausführungsform wird ein Katalysator auf Basis von Platin und Silber eingesetzt. Vorzugsweise besteht der Katalysator aus Platin und Silber auf einem Trägermaterial. Das Trägermaterial kann aus der Gruppe bestehend aus Siliciumdioxid, Alumosilikat, Aluminiumoxid, Kieselgel, Aktivkohle, Titandioxid und Mischungen daraus, vorzugsweise aus der Gruppe bestehend aus Siliciumdioxid, Aluminiumoxid, Titandioxid und Mischungen daraus ausgewählt werden. Das Platin liegt vorzugsweise in einer Menge von 0,01 bis 1 Gew.-%, vorzugsweise 0,05 bis 0,5 Gew.-%, besonders bevorzugt 0,1 bis 0,3 Gew.-%, bezogen auf das Gesamtgewicht des Katalysators vor. Das Silber liegt vorzugsweise in einer Menge von 0,01 bis 1 Gew.-%, vorzugsweise 0,05 bis 0,5 Gew.-%, besonders bevorzugt 0,1 bis 0,3 Gew.-%, bezogen auf das Gesamtgewicht des Katalysators vor.

Der heterogene Katalysator für die Selektivhydrierung besteht vorzugsweise aus Katalysatorpartikeln, insbesondere als Granulat, in Form von Extrudaten oder in Form von Kugeln, und ist somit kein monolithisches Material.

Die Selektivhydrierung in Schritt (a) kann weiterhin bevorzugt in Anwesenheit von Kohlenmonoxid (CO) als Moderator erfolgen. Der Gehalt an Kohlenmonoxid bei der Selektivhydrierung beträgt vorzugsweise zwischen 0,05 und 20 ppm Kohlenmonoxid, bezogen auf die gesamte Masse des C4-Kohlenwasserstoffstroms. Bevorzugt werde zwischen 0,5 und 5 ppm Kohlenmonoxid zugegeben.

Die Selektivhydrierung erfolgt in Schritt a) in einer Hydrierzone, die mindestens einen Reaktor R1 umfasst. Der Reaktor R1 kann jeder geeignete Reaktor sein, beispielsweise ein Rieselbettreaktor, ein Kreislaufreaktor oder ein Pulse-Flow-Reaktor. Entsprechende Reaktoren sind dem Fachmann bekannt. In einer bevorzugten Ausführungsform wird der Reaktor R1 in Kreislauffahrweise betrieben und ein Teil des hydrierten Stroms nach dem Kühlen in Schritt b) zurück zum Reaktor R1 geführt.

In einer bevorzugten Ausführungsform weist die Hydrierzone einen weiteren Reaktor R2 auf, der mit einem Teil des hydrierten Stroms nach dem Kühlen in Schritt b) und einem Wasserstoff-haltigen Gas, vorzugsweise Wasserstoff gespeist wird, sodass im Reaktor R2 ebenfalls eine Selektivhydrierung von 1,3-Butadien unter Erhalt des hydrierten Stroms stattfindet. Der hydrierte Strom enthält 1,3-Butadien vorzugsweise in einer Menge von < 2500 Massen-ppm.

In der vorstehenden genannten bevorzugten Ausführungsform der vorliegenden Erfindung wird die Selektivhydrierung in Schritt a) also in mindestens zwei Reaktoren R1 und R2 durchgeführt. Besonders bevorzugt wird die Selektivhydrierung in Schritt a) in mindestens einem Kreislaufreaktor und in mindestens einem Reaktor, der im geraden Durchgang betrieben wird, durchgeführt. Der Kreislaufreaktor dabei der erste Reaktor R1 und der im geraden Durchgang betriebene Reaktor der zweite Reaktor R2.

### Schritt b)

Der aus Schritt a) erhaltenen hydrierte Strom wird in Schritt b) gekühlt. Das Kühlen des hydrierten Stroms aus dem Reaktor R1 und/oder, sofern vorhanden, dem Reaktor R2 erfolgt in einem Wärmetauscher WT1, wobei Energie in Form von Wärme vom hydrierten Strom auf einen flüssigen oder gasförmigen Wärmeträger W übertragen wird, wodurch ein Wärmeträger W1 entsteht.

Durch die Übertragung von Energie auf den Wärmeträger W wird sich der Energiegehalt des hydrierten Stroms verringern, der hydrierte Strom sich also abkühlen und/oder kondensieren. In Schritt b) wird also die Kondensationsenergie des hydrierten Stroms ausgenutzt, um Energie in Form von Wärme auf einen Wärmeträger zu übertragen und dadurch für die Erzeugung von Dampf nutzbar zu machen.

Als Wärmeträger W kann jedes dem Fachmann geläufige Arbeitsmedium genutzt werden. Der Wärmeträger W wird vorzugsweise aus der Gruppe bestehend aus Wasser; Alkoholen; Alkohol-Wasser-Mischungen; Salz-Wasser-Lösungen; Ammoniak; Mineralölen, wie zum Beispiel Dieselölen; Thermalölen, wie zum Beispiel Silikonölen; biologische Ölen, wie zum Beispiel Limonen; und aromatischen oder aliphatischen Kohlenwasserstoffen, wie zum Beispiel Dibenzyltoluol ausgewählt, weiterhin bevorzugt ist der Wärmeträger W Methanol, Ethanol, Propanol, n-Pentan, n-Butan, n-Hexan, n-Propan oder Ammoniak, besonders bevorzugt n-Pentan.

Wird der Wärmeträger W in Schritt b) in flüssiger Phase, also ein flüssiger Wärmeträger W, eingesetzt und diesem in Schritt b) Energie, bevorzugt Wärme, zugeführt, wird der Wärmeträger W zumindest teilweise verdampft und dadurch ein gasförmiger Wärmeträger W1 erhalten. Wird der Wärmeträger W in Schritt b) in gasförmiger Phase, also ein gasförmiger Wärmeträger W eingesetzt und diesem in Schritt b) Energie, bevorzugt Wärme, zugeführt, wird ebenfalls ein gasförmiger Wärmeträger W1 erhalten.

Mit der Formulierung "flüssiger Wärmeträger" ist im Rahmen der vorliegenden Erfindung gemeint, dass > 50 Gew.-%, weiterhin bevorzugt > 55 Gew.-%, weiterhin bevorzugt > 75 Gew.-%, weiterhin bevorzugt > 90 Gew.-% und besonders bevorzugt > 99 Gew.-% des in Schritt b) eingesetzten Wärmeträgers im flüssigen Aggregatzustand vorliegen, jeweils bezogen auf das Gesamtgewicht des in Schritt b) eingesetzten Wärmeträgers.

Mit der Formulierung "gasförmiger Wärmeträger" ist im Rahmen der vorliegenden Erfindung gemeint, dass > 30 Gew.-%, weiterhin bevorzugt > 50 Gew.-%, weiterhin bevorzugt > 75 Gew.-%, weiterhin bevorzugt > 90 Gew.-% und besonders bevorzugt > 99 Gew.-% des in Schritt (c) eingesetzten Wärmeträgers im gasförmigen Aggregatzustand vorliegen, jeweils bezogen auf das Gesamtgewicht des in Schritt b) eingesetzten Wärmeträgers.

Die Übertragung von Energie in Schritt b) kann nach dem Fachmann bekannten Verfahren oder mit dem Fachmann bekannten Wärmetauschern, beispielsweise mit sogenannten Verdampfern, durchgeführt werden. Geeignete Verdampfer, die als Wärmetauscher eingesetzt werden können, sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Neben den genannten kann aber auch jede beliebige andere, dem Fachmann bekannte Verdampferbauart, die sich zum genannten Einsatz eignet, eingesetzt werden.

In der allgemeinen Ausführungsform wird der hydrierte Strom aus dem Reaktor R1 gekühlt und zur Wärmeintegration bzw. der Dampferzeugung genutzt. Im Rahmen der vorliegenden Erfindung ist es jedoch, wie erwähnt, bevorzugt, dass die Hydrierzone mindestens zwei Reaktoren R1 und R2 umfasst, die in Reihe geschaltet vorliegen. Es ist demnach bevorzugt, wenn die Hydrierzone einen weiteren Reaktor R2 aufweist, der mit einem Teil des hydrierten Stroms optional nach Kühlung und einem Wasserstoff-haltigen Gas, vorzugsweise Wasserstoff gespeist wird, sodass im Reaktor R2 eine Selektivhydrierung von 1,3-Butadien unter Erhalt eines Produktstroms stattfindet. Die Merkmale der Selektivhydrierung entsprechen vorzugsweise den unter Schritt a) genannten Merkmalen der Selektivhydrierung, insbesondere im Hinblick auf die Reaktionsbedingungen wie Temperatur und Druck, des Aggregatzustands während der Reaktion oder des eingesetzten Katalysators. Der zweite Reaktor R2 wird vorzugsweise im geraden Durchgang betrieben.

Die Kühlung in Schritt b) kann bei Vorliegen von zwei Reaktoren R1 und R2 auf zwei unterschiedliche Wege erfolgen:
In einer Ausführungsform wird nur der aus dem Reaktor R2 erhaltene hydrierte Strom gemäß Schritt b) gekühlt. Die beschriebene Verfahrensweise gemäß Schritt b) bleibt gleich, d. h. der hydrierte Strom aus dem Reaktor R2 wird genutzt, um den Wärmeträger W zu erhitzen und kühlt dabei ggf. unter Kondensation ab.

In einer weiteren Ausführungsform kann sowohl der aus dem Reaktor R1 erhaltene hydrierte Strom als auch der aus dem Reaktor R2 erhaltene Strom gemäß Schritt b) gekühlt werden. In diesem Fall bleibt die Kühlung des hydrierten Stroms aus dem Reaktor R1 durch den Wärmeaustausch mit dem Wärmeträger W erhalten. Zusätzlich dazu wird auch der hydrierte Strom aus dem Reaktor R2 gekühlt. Das Kühlen des hydrierten Stroms aus dem Reaktor R2 erfolgt vorzugsweise in einem Wärmetauscher WT2, wobei Energie in Form von Wärme vom hydrierten Strom auf einen flüssigen oder gasförmigen Wärmeträger P übertragen wird, wodurch ein Wärmeträger P1 entsteht.

Als Wärmeträger P kann jedes dem Fachmann geläufige Arbeitsmedium genutzt werden. Der Wärmeträger P wird vorzugsweise aus der Gruppe bestehend aus Wasser; Alkoholen; Alkohol-Wasser-Mischungen; Salz-Wasser-Lösungen; Ammoniak; Mineralölen, wie zum Beispiel Dieselölen; Thermalölen, wie zum Beispiel Silikonölen; biologische Ölen, wie zum Beispiel Limonen; und aromatischen oder aliphatischen Kohlenwasserstoffen, wie zum Beispiel Dibenzyltoluol ausgewählt, weiterhin bevorzugt ist der Wärmeträger W Methanol, Ethanol, Propanol, n-Pentan, n-Butan, n-Hexan, n-Propan oder Ammoniak, besonders bevorzugt n-Pentan.

Es ist bevorzugt, wenn die Wärmeträger W und P zu einem gemeinsamen Wärmeträgerkreislauf gehören. Deshalb sind die Wärmeträger W und P vorzugsweise identisch. Besonders bevorzugt ist es, wenn die beiden Wärmeträger W1 und P1 vor der Verdichtung in Schritt c) vermischt werden. Der vermischte Wärmeträger wird in diesem Fall der Einfachheit halber weiter als W1 bezeichnet werden.

Die Übertragung von Energie im WT2 kann nach dem Fachmann bekannten Verfahren oder mit dem Fachmann bekannten Wärmetauschern, beispielsweise mit sogenannten Verdampfern, durchgeführt werden. Geeignete Verdampfer für den Einsatz als WT2 sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Neben den genannten kann aber auch jede beliebige andere, dem Fachmann bekannte Verdampferbauart, die sich zum genannten Einsatz eignet, eingesetzt werden.

Der hydrierte Strom wird nach dem Kühlen in Schritt (b) vorzugsweise zu einem Flashbehälter geführt wird, wo Leichtsieder mittels Flash entfernt werden. Anschließend wird der hydrierte Strom ausgeschleust und der weiteren Verarbeitung zugeführt, z. B. eine 1-Butenabtrennung oder einer Oligomerisierung.

### Schritt c)

Im anschließenden Schritt c) wird mindestens ein Teil des Wärmeträgers W1, vorzugsweise der gesamte Wärmeträger W1, verdichtet, wodurch ein gegenüber W1 verdichteter Wärmeträger W2 entsteht, der einen höheren Druck aufweist als der Wärmeträger W1. Der Druck von W2 liegt nach dem Verdichten vorzugsweise im Bereich von 1 bar bis 30 bar, bevorzugt 3 bar bis 15 bar auf.

Das Verdichten des mindestens einen Teils des Wärmeträgers W1 in Schritt c) kann auf jede beliebige, dem Fachmann bekannte Art erfolgen. So kann die Verdichtung zum Beispiel mechanisch und einstufig oder mehrstufig durchgeführt werden. Einstufig bedeutet in diesem Zusammenhang, dass eine Verdichtung von einem auf ein anderes Druckniveau stattfindet. Mehrstufig bedeutet, dass erst auf ein Druckniveau X und dann von X auf das Druckniveau Y verdichtet wird. Bei einer mehrstufigen Verdichtung können mehrere Verdichter gleicher Bauart oder Verdichter unterschiedlicher Bauart eingesetzt werden. Eine mehrstufige Verdichtung kann mit einer oder mehreren Verdichtermaschinen erfolgen. Der Einsatz einer einstufigen Verdichtung oder einer mehrstufigen Verdichtung ist vom Verdichtungsverhältnis und somit davon abhängig, auf welchen Druck der Wärmeträger W1 verdichtet werden soll.

In einer bevorzugten Ausführungsform der vorliegenden Erfindung erfolgt die Verdichtung mehrstufig. Dabei wird der Wärmeträger W1 in zwei Verdichterstufen V1 und V2 verdichtet, wobei in der ersten Verdichtung ein verdichteter Wärmeträger W1.1 entsteht, der einen höheren Druck als W1 aufweist und in der zweiten Verdichtung der verdichtete Wärmeträger W2 entsteht, der einen höheren Druck als W1.1 und als W1 aufweist.

Als Verdichter im erfindungsgemäßen Verfahren, insbesondere zum Verdichten des Wärmeträgers W1 in Schritt c), eignet sich jeder beliebige, dem Fachmann bekannte Verdichter, bevorzugt mechanische

Verdichter, mit dem sich Gasströme verdichten lassen. Geeignete Verdichter sind zum Beispiel ein- oder mehrstufige Getriebeturboverdichter, Kolbenverdichter, Schraubenverdichter, Zentrifugalverdichter oder Axialverdichter.

### Schritt d)

Der sich anschließende Schritt d) betrifft dann die Wärmeübertragung. Dabei wird Energie vom verdichteter Wärmeträger W2 auf einen weiteren Wärmeträger D übertragen, wodurch ein Dampfstrom erzeugt wird. Der Dampfstrom kann dann an anderer Stelle im Verfahren oder an einer anderen Produktionsanlage zur Beheizung eingesetzt werden.

Als Wärmeträger D können grundsätzlich eine große Zahl an Wärmeträgern eingesetzt werden. In der Regel wird an chemischen Produktionsstandorten jedoch Wasserdampf zur Beheizung eingesetzt. Der Wärmeträger D ist deshalb vorzugsweise Wasser und der durch die Energieübertragung in Schritt d) erhaltene Dampf ist dementsprechend vorzugsweise Wasserdampf. Der in Schritt d) erzeugte Dampfstrom ist somit vorzugsweise ein Wasserdampfstrom. Der so erzeugte Dampf, vorzugsweise Wasserdampf, also der Dampfstrom weist nach der Energieübertragung in Schritt d) vorzugsweise einen Druck im Bereich von 1,2 bis 6 bar, besonders bevorzugt im Bereich von 2,2 bis 4,5 bar auf.

Die Energieübertragung in Schritt d) von W2 auf den weiteren Wärmeträger D erfolgt in einem Verdampfer. Geeignete Verdampfer, die in Schritt d) eingesetzt werden können, sind zum Beispiel Naturumlaufverdampfer, Zwangsumlaufverdampfer, Zwangsumlaufverdampfer mit Entspannung, Kesselverdampfer, Kettleverdampfer, Fallfilmverdampfer oder Dünnschichtverdampfer. Neben den genannten kann aber auch jede beliebige andere, dem Fachmann bekannte Verdampferbauart, die sich zum genannten Einsatz eignet, eingesetzt werden.

Mit zumindest einem Teil des so erzeugten Dampfstroms, vorzugsweise mit zumindest einem Teil des Wasserdampfes, werden in einer bevorzugten Ausführungsform der vorliegenden Erfindung eine oder mehrere Destillationskolonnen beheizt. Es ist weiterhin bevorzugt, wenn die Destillationskolonne nicht zur Selektivhydrierung gemäß Schritt a) gehört.

Die Destillationskolonne, die mit dem in Schritt d) erzeugten Dampfstrom beheizt wird, wird vorzugsweise bei der Abtrennung von 1-Buten, bei der Abtrennung von Leichtsiedern innerhalb einer Butadienabtrennung, bei der Acetylenabtrennung in einer Butadiendienanlage, die Reindestillation in einer Butadienanlage, als eine MTBE-Kolonne oder als Desorber in einer Butadienanlage eingesetzt. Die Destillationskolonnen werden vorzugsweise über sogenannte Sumpfverdampfer beheizt, bei denen ein Teil des Sumpfs durch den Sumpfverdampfer geführt, erhitzt und dann wieder in die Destillationskolonne zurückgeführt wird.

Der Wärmeträger W2 wird bei der Energieübertragung in Schritt d) vorzugsweise zumindest teilweise kondensiert. Das Kondensat wird dann als W zur Wärmeübertragung zurückgeführt und durch die Wärmeübertragung von W4 erneut verdampft.

Im Falle einer mehrstufigen Verdichtung, wo in Schritt c) die beiden Verdichtungsstufen V1 und V2 vorliegen, kann der zumindest teilweise kondensierte Wärmeträger W2 alternativ vorher in einen Flashbehälter gefahren werden, der auf dem Druckniveau betrieben wird, das zwischen den beiden Verdichtungsstufen V1 und V2 liegt, wodurch in dem Flashbehälter eine gasförmige Phase und eine flüssige Phase anfallen.

In einer bevorzugten Ausführungsform wird der Flashanteil im Flashbehälter als Gasphase zwischen die beiden Verdichtungsstufen V1 und V2 geleitet. Dadurch kann ein Teil der nach der Energieübertragung in Schritt d) noch im Wärmeträger W2 vorhandenen Energie genutzt werden. Der Flashanteil wird mit dem verdichteten Wärmeträger W1.1 vermischt, der zweiten Verdichtung V2 unterworfen und zur Energieübertragung in Schritt d) geführt. Nur der flüssige Anteil im Flashbehälter wird dann vorzugsweise als Wärmeträger W zu Schritt b) geleitet und durchläuft die dann die weiteren Schritte. Der flüssige Anteil kann weiterhin zur Vorwärmung des Wärmeträgers W1 vor der Verdichtung V1 eingesetzt wird, wobei Energie vom flüssigen Anteil auf den Wärmeträger W1 übertragen wird. Der Wärmeträger W1 wird dadurch überhitzt und eine Kondensation bei der Verdichtung verhindert.

### Abbildungen

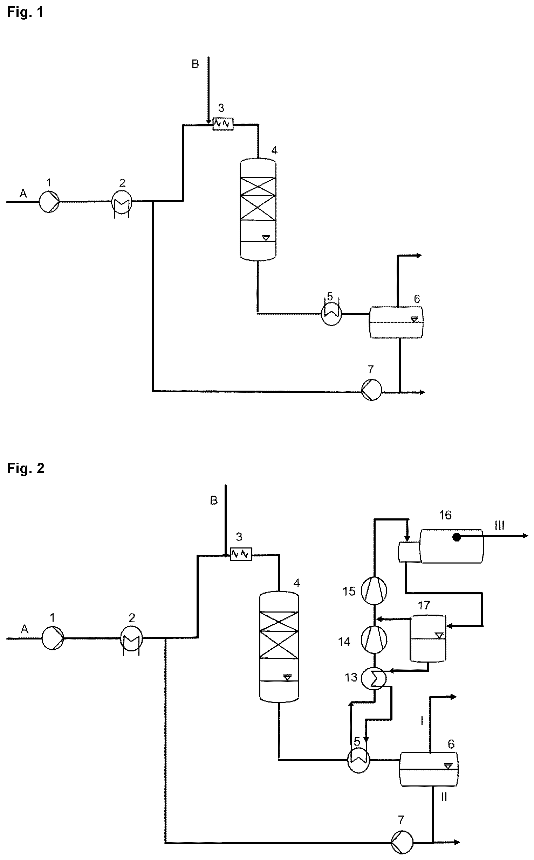

Fig. 1 zeigt eine nicht erfindungsgemäße Ausführungsform der vorliegenden Erfindung. Der C4-Kohlenwasserstoffstrom (A) über eine Druckerhöhungspumpe (1) und einem Vorwärmer (2) zu einem Reaktor (4) gefahren. Zusammen mit dem C4-Kohlenwasserstoffstrom (A) wird Wasserstoff (B) dem Reaktor zugeführt. Vorher werden die Ströme (A, B) in einem Mischer (3), beispielsweise einem statischen Mischer, vermischt. Im Reaktor (4) erfolgt die selektive Hydrierung von 1,3 Butadien zu linearen Butenen. Das aus dem Reaktor (4) abgeführte hydrierter Reaktionsgemisch wird über den Wärmeübertrager (5) gekühlt. Der Strom wird einem Flashbehälter (6) zugeführt. Der nicht gelöste Wasserstoff (I) wird abgezogen und kann beispielsweise einem Sammelgasstrom zugeführt. Der Großteil des restlichen Reaktionsgemisches (II) wird über eine zweite Druckerhöhungspumpe (7) als Kreislauf dem Feed (A) zugemischt. Ein weiterer Teil wird ausgeschleust und kann beispielsweise zu einem zweiten Hydrierreaktor zugeführt werden.

Fig. 2 zeigt eine erfindungsgemäße Ausführungsform der vorliegenden Erfindung. Im Unterschied zu der Ausführungsform nach Fig. 1 weist die erfindungsgemäße Ausführungsform gemäß Fig. 2 eine Vorrichtung zur Erzeugung von Heizdampf auf. Im Wärmeübertrager (5) wird die Reaktionswärme auf den Wärmeträger übertragen. Der Wärmeträger wird einer Überhitzung (13) unterzogen und anschließend mehrstufig verdichtet (14, 15). Mit dem überhitzten und verdichteten Wärmeträger wird in einem Verdampfer (16) der Heizdampfstrom (III) erzeugt. Der Dampfstrom könnte einer weiteren Verdichtung unterzogen werden und auf ein höheres Druckniveau gebracht werden (nicht in der Abbildung gezeigt). Der aus dem Verdampfer (16) wieder austretende Wärmeträger, von dem Energie übertragen worden ist, wird zurückgeführt. Dabei wird zunächst der Wärmeträger in einen Zwischenentspannungsbehälter (17) gefahren, der bei einem Druck zwischen den beiden Verdichtungsstufen (14, 15) betrieben wird. Ein Teil des Wärmeträgers wird zwischen die beiden Verdichtungsstufen zugeführt, der restliche Teil wird zum Wärmeübertrager (5) zurückgeführt.

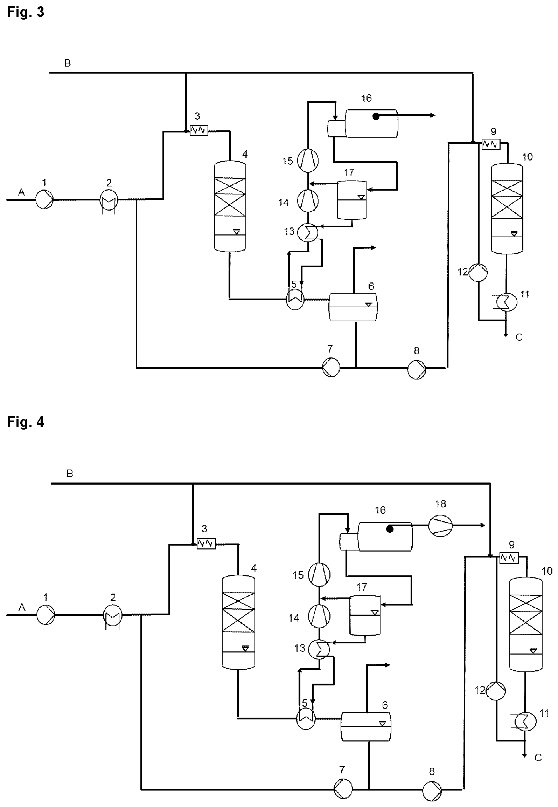

Fig. 3 zeigt eine erfindungsgemäße Ausführungsform. Der Unterschied zu Fig. 2 ist die zweite Reaktionsstufe. Dabei wird ein Teil des restlichen Reaktionsgemisches (II) über eine dritte Druckerhöhungspumpe (8) zu einem zweiten Reaktor (10) geführt. Vorher wird der Strom mit Wasserstoff in einem zweiten Mischer (9) vermischt. Das Reaktionsgemisch aus dem zweiten Reaktor (10) wird in einem Kühler (11) gekühlt, teilweise als Produktstrom (C) abgeführt und teilweise über eine vierte Druckerhöhungspumpe zurückgeführt.

Fig. 4 zeigt eine weitere erfindungsgemäße Ausführungsform. Der Unterschied zu Fig. 3 besteht darin, dass der entstandene Heizdampf verdichtet (18) wird.

### Beispiele:

Alle Beispiele wurden mit Aspen Plus 12 mit einem mit Betriebsversuchen validierten Modell berechnet. Für alle Simulationen wurde ein Feedstrom mit einer Zusammensetzung von 37 Gew.-% 1,3-Butadien, 24,5 Gew.-% Isobuten, 16,6 Gew.-% n-Butan, 9,8 Gew.-% 1-Buten, 5,4 Gew.-% trans-Buten, 3,2 Gew.-% cis-Buten, 3,5 Gew.-% Isobutan betrachtet.

### Beispiel 1

29 t/h des oben beschriebenen Feedstroms (A) werden gemäß der in Fig. 1 beschriebenen Ausführungsform behandelt. Für die Hydrierung wird Wasserstoff (B) mit einem 10%-stöchiometrischen Überschuss zugeführt. Im Reaktor findet bei einer Feedtemperatur von 45°C die selektive Hydrierung von 1,3 Butadien zu linearen Butenen statt. Die Reaktionswärme von 5 MW wird über den Wärmeübertrager (5) abgeführt. Der Großteil des Reaktionsgemisches (435t/h) wird als Kreislauf dem Feed (A) zugemischt. 29 t/h werden einer weiteren Reaktionsstufe (nicht gezeigt) zugeführt.

### Beispiel 2

In dieser Ausführung gemäß Fig. 2 oder Fig. 3 wird die Reaktionswärme genutzt und mit einer mehrstufigen Wärmepumpe mit Zwischenentspannung Dampf erzeugt, der in anderen Teilen einer Verbundanlage eingesetzt wird. Im Wärmeübertrager (5) wird die Reaktionswärme an den Wärmeträger n-Pentan übertragen. Der Wärmeträger verdampft bei 1,2 bar absolut / 40°C und wird einer Überhitzung (13) von 40 K unterzogen. Anschließend wird der überhitzte Strom verdichtet und bei einem höheren Druckniveau von 12,1 bar im Verdampfer (16) kondensiert, wodurch der Heizdampfstrom mit einem Druck von 2,5 bar entsteht. Für den Vorgang des Verdichtens wird eine elektrische Leistung von 2,0 MW benötigt. Es wird insgesamt eine thermische Leistung von 7 MW über einen 2,5 bar Dampfstrom bereitgestellt. Demzufolge errechnet sich ein COP von 3,5.

### Bezugszeichenliste

- A: C4-Kohlenwasserstoffstrom
- B: Wasserstoffstrom
- C: Produktstrom
- 1: Druckerhöhungspumpe
- 2: Vorwärmer
- 3: Mischer
- 4: Reaktor
- 5: Wärmeübertrager
- 6: Flashbehälter
- 7: zweite Druckerhöhungspumpe
- 8: dritte Druckerhöhungspumpe
- 9: zweiter Mischer
- 10: zweiter Reaktor
- 11: Kühler
- 12: vierte Druckerhöhungspumpe
- 13: Überhitzer
- 14: Verdichter
- 15: Verdichter
- 16: Verdampfer
- 17: Zwischenentspannungsbehälter
- 18: Verdichter
- I: nicht gelöster Wasserstoff
- II: restliches Reaktionsgemisch

## Patentansprüche

1. Verfahren zur Erzeugung eines Dampfstroms, wobei das Verfahren die folgenden Schritte umfasst
a) Zuführen eines C4-Kohlenwasserstoffstroms, der zumindest 1,3-Butadien, 1-Buten, 2-Buten und Isobutan enthält, und Zuführen eines Wasserstoff-haltigen Gases, vorzugsweise Wasserstoff zu einer Selektivhydrierung, um zumindest einen Teil des enthaltenen 1,3 Butadiens in Anwesenheit eines heterogenen Katalysators zu hydrieren, wobei die Selektivhydrierung in einer Hydrierzone erfolgt, die mindestens einen Reaktor R1 umfasst und wobei aus dem Reaktor R1 ein hydrierter Strom entnommen wird;
b) Kühlen des hydrierten Stroms aus dem Reaktor R1 in einem Wärmetauscher WT1, wobei Energie in Form von Wärme vom hydrierten Strom auf einen flüssigen oder gasförmigen Wärmeträger W übertragen wird, wodurch ein Wärmeträger W1 entsteht;
c) Verdichten mindestens eines Teils des Wärmeträgers W1, vorzugsweise des gesamten Wärmeträgers W1, wodurch ein verdichteter Wärmeträger W2 entsteht, der einen höheren Druck aufweist als der Wärmeträger W1; und
d) Übertragen von Energie vom verdichteter Wärmeträger W2 auf einen weiteren Wärmeträger D übertragen wird, wodurch der herzustellende Dampfstrom erzeugt wird.

2. Verfahren nach Anspruch 1, wobei der Wärmeträger W aus der Gruppe, bestehend aus Wasser; Alkoholen; Alkohol-Wasser-Mischungen; Salz-Wasser-Lösungen; Ammoniak; Mineralöle, wie zum Beispiel Dieselöle; Thermalölen, wie zum Beispiel Silikonöle; biologische Ölen, wie zum Beispiel Limonen; und aromatischen oder aliphatischen Kohlenwasserstoffen, wie zum Beispiel Dibenzyltoluol ausgewählt wird, vorzugsweise Wasser, Methanol, Ethanol, Propanol, n-Pentan, n-Butan, n-Hexan, n-Propan oder Ammoniak, besonders bevorzugt n-Pentan ist.

3. Verfahren nach Anspruch 1 oder 2, wobei der Wärmeträger D Wasser ist.

4. Verfahren nach einem der vorhergehenden Ansprüche, wobei der erzeugte Dampfstrom zur Beheizung einer oder mehrere Destillationskolonnen eingesetzt wird.

5. Verfahren nach einem der vorhergehenden Ansprüche, wobei der in Schritt (d) erzeugte Dampf einen Druck im Bereich von 2 bis 5 bar aufweist.

6. Verfahren nach einem der vorhergehenden Ansprüche, wobei der Wasserstoff und der C4-Kohlenwasserstoffstrom vor dem Zuführen in den Reaktor R1 in einem statischen Mischer vermischt werden.

7. Verfahren nach einem der vorhergehenden Ansprüche, wobei die Hydrierzone einen weiteren Reaktor R2 aufweist, der mit einem Teil des hydrierten Stroms optional nach Kühlung und einem Wasserstoff-haltigen Gas, vorzugsweise Wasserstoff gespeist wird, sodass im Reaktor R2 eine Selektivhydrierung von 1,3-Butadien unter Erhalt eines Produktstroms stattfindet.

8. Verfahren nach Anspruch 7, wobei nur der aus dem Reaktor R2 erhaltene hydrierte Strom gemäß Schritt b) im Wärmetauscher WT2 gekühlt wird, wobei Energie in Form von Wärme vom aus dem Reaktor R2 erhaltenen hydrierten Strom auf einen flüssigen oder gasförmigen Wärmeträger W übertragen wird, wodurch ein Wärmeträger W1 entsteht.

9. Verfahren nach Anspruch 7, wobei sowohl der aus dem Reaktor R1 erhaltene hydrierte Strom als auch der aus dem Reaktor R2 erhaltene Strom gemäß Schritt b) gekühlt werden, wobei die Kühlung des hydrierten Stroms aus dem Reaktor R2 in einem Wärmetauscher WT2, wobei Energie in Form von Wärme vom hydrierten Strom auf einen flüssigen oder gasförmigen Wärmeträger P übertragen wird, wodurch ein Wärmeträger P1 entsteht.

10. Verfahren nach Anspruch 9, wobei die beiden Wärmeträger W1 und P1 vor der Verdichtung in Schritt c) vermischt werden.

11. Verfahren nach einem der vorhergehenden Ansprüche, wobei der Reaktor R2 in Kreislauffahrweise betrieben wird und ein Teil Produktstroms nach dem Kühlen in Schritt zurück zum Reaktor R2 geführt wird.

12. Verfahren nach einem der vorhergehenden Ansprüche, wobei der Wärmeträger W2 bei der Energieübertragung in Schritt d) zumindest teilweise kondensiert.

13. Verfahren nach einem der vorhergehenden Ansprüche, wobei die Hydrierung in Schritt a) in flüssiger Phase erfolgt.

14. Verfahren nach einem der vorhergehenden Ansprüche, wobei die Hydrierung in Schritt a) an einem Palladium- und/oder Platin-haltigen Festbettkatalysator erfolgt.

15. Verfahren nach Anspruch 14, wobei der Katalysator 0,1 bis 2 Massen-% Palladium und/oder Platin auf einem Träger aufweist.
